(19) Europäisches Patentamt
European Patent Office
Office européen des brevets

(11) Publication number: **0 067 476**
**A2**

# (12) EUROPEAN PATENT APPLICATION

(21) Application number: 82200673.0

(22) Date of filing: 02.06.82

(51) Int. Cl.$^3$: A 61 K 7/16

(30) Priority: 11.06.81 DE 3123211

(43) Date of publication of application:
22.12.82 Bulletin 82/51

(84) Designated Contracting States:
AT BE CH DE FR GB IT LI NL SE

(71) Applicant: UNILEVER NV
Burgemeester s'Jacobplein 1 P.O. Box 760
NL-3000 DK Rotterdam(NL)

(84) Designated Contracting States:
BE CH DE FR IT LI NL SE AT

(71) Applicant: UNILEVER PLC
Unilever House Blackfriars P O Box 68
London EC4P 4BQ(GB)

(84) Designated Contracting States:
GB

(72) Inventor: Bandzauner, Arnold, Dr.
Olendeelskoppel 16
D-2000 Hamburg 65(DE)

(72) Inventor: Forsthoff, Ernst Ludwig, Dr.
Baumschulenweg 14
D-2000 Hamburg-Schenefeld(DE)

(72) Inventor: Ernerth, Martin, Dr.
Quellgrund 18b
D-2014 Hamburg 92(DE)

(74) Representative: Van Gent, Jan Paulus et al,
Unilever N.V. Patent Division P.O. Box 137
NL-3130 AC Vlaardingen(NL)

## (54) Dentifrice composition.

(57) The inclusion of epigallo-catechin-3-gallate or a polyvalent metal salt, such as the zinc salt, of glycyrrhizinic acid in a dentifrice composition provides a dentifrice with a keratinisation-promoting effect, which reduces gingivitis.

EP 0 067 476 A2

Croydon Printing Company Ltd.

# DENTIFRICE COMPOSITION

The present invention relates to dentifrice compositions comprising certain additives affecting the keratinisation of the human gingiva.

The sound human gingiva is protected on the surface by a horny layer of coreless little scales against exogenic influences, particularly of bacterial origin. In the sulcus this keratinized layer is lacking, so that the metabolic products of bacteria, e.g. of plaque bacteria, can penetrate from there into the parodontium, which can cause gingivitis, resulting, on further progress, in paradontosis and loosening of teeth. On inflammation of the gums the formation of core-containing non-keratinized cells is accelerated; they are conveyed to the surface of the gingiva, where they reduce the so-called keratinisation index, i.e. the amount of protective horny scales. The susceptibility of the gingiva to all kinds of exogenic irritations is thus further increased.

Since caries and paradontosis are world-wide epidemics, for a long time a great variety of therapeutic or prophylactic active ingredients has been suggested or applied to reduce or remedy these defects, which are attributable to insufficient oral hygiene. The addition of such active ingredients to dentifrices, such as mouth washes, toothpastes, toothpowders, mouth tablets,etc. is also general practice. Typical examples of such active ingredients are vitamins, anti-inflammatory agents, perfusion-promoting agents, adstringents, antibiotics, etc. These ingredients include tartaric acid, urea, allantoin, pyridyl carbinol tartrate, D-panthenol, alpha-tocopherol, azulene, nicotinic acid amide, etc. However, as to their effectiveness, decisive evidence is often lacking, or relatively slight and only of a temporary nature.

The object of the present invention was to find active ingredients which, when added to a dentifrice composition, can exert a permanent keratinising action on the intra-oral mucosa.

This object was attained in that it was found that epigallo-catechin-3-gallate and zinc glycyrrhizinate exert such a desirable keratinising effect on the intra-oral mucosa.

The invention therefore relates to dentifrice compositions having a keratinisation-promoting effect on the intra-oral mucosa, characterized in that they contain an effective amount of epigallo-catechin-3-gallate and/or salts of glycyrrhizinic acid and polyvalent metals, such as Al, Sn, Mg and Zn, preferably zinc glycyrrhizinnate.

The content of active ingredients in the dentifrice compositions according to the invention is generally from 0.1 to 10 percent by weight, preferably from 0.5 to 5 percent by weight, calculated on the finished dentifrice composition. The dentifrice compositions further comprise the constituents usually incorporated in all kinds of dentifrice compositions as they are known from the prior art for mouthwashes, toothpastes, toothpowders, mouth tablets, etc., e.g. from the "Handbuch der Kosmetika und Riechstoffe", Vol.3, 1973, pages 777 ff, by H. Janistyn.

The epigallo-catechin-3-gallate is a substance known per se, having the following structural formula:

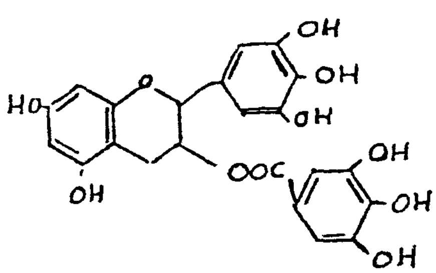

This compound can be prepared from gallic acid and catechin; however, it is also found in nature, e.g. in green tea, and can be obtained therefrom.

Zinc glycyrrhizinate is the zinc salt of glycyrrhizinic acid; this acid is the glycoside of glycyrrhetinic acid. Glycyrrhizinic acid is found in nature, particularly in licorice-roots, from which it is obtained by means of extraction. The zinc salt is obtained by reaction of the acid with a suitable zinc compound, such as zinc chloride, zinc hydroxide, zinc sulphate or zinc citrate.

The epigallo-catechin-3-gallate is readily water-soluble in the concentration to be used, whereas the zinc glycyrrhizinate is not. The latter remains in suspension as a white powder,but it can be finely dispersed in oily carriers, or suspended or dissolved in (hydro)alcoholic solutions.

The zinc glycyrrhizinate can be prepared separately and then incorporated into the dentrifice composition; it can however also be formed in situ in the dentrifice composition, if glycyrrhizinic acid or an alkalimetal- or ammonium-salt thereof and a suitable zinc compound, e.g. zinc citrate, are added to the dentifrice composition.

Example 1

For experiments on the human gingiva an exfoliative-cytological investigation was carried out with five testpanels each comprising 2 persons (a man and a woman) over a period of 7 days. As test products the following compositions were used:

Group A: a conventional toothpaste on the basis of aluminiumoxide trihydrate as abrasive, with titanium dioxide, polysaccharide, sodiumbenzoate, sodiumlaurylsulphate, flavours, sorbitol and water;

Group B: a conventional toothpaste based on silica gel as abrasive, with titanium dioxide, CMC, polyethylene glycol, sodiumlaurylsulphate, flavours, sorbitol, water and 1% of sodiummonofluorophosphate.

Group C: Basis as for A, but with addition of 4.5% of zinc glycyrrhizinate and 0.8% of sodiummonofluorophosphate;

Group D: an aqueous solution of 0.5% of epigallo-catechin-3-gallate;

Group E: an aqueous solution of 0.75% of epigallo-cathechin-3-gallate.

The toothpastes of Groups A and B were current toothpastes and were used as placebos.

The test-panellists brushed their teeth twice daily for 2 minutes with one of the toothpastes A - C. In Groups D and E, in addition to brushing with toothpaste A, the solutions were allowed to act on the gingiva for 1 minute by means of a little sponge. Each day cell scrapings were taken from the right-hand top section of the teeth, which were evaluated cytologically. After the usual colouring according to the method of Papanicolaou the keratinisation index (K.I.) was determined; this indicates the proportional ratio of coreless horny scales to the total amount of the cells.

The following results were obtained:

| Tooth-paste/ solution | Keratinisation Index | | | | | | | |
|---|---|---|---|---|---|---|---|---|
| | Start | 1st day | 2nd day | 3rd day | 4th day | 5th day | 6th day | 7th day |
| A | 7 | 9 | 5 | 5 | 6 | 10 | 7 | 5 |
| B | 35 | 30 | 22 | 30 | 23 | 18 | 27 | 25 |
| C | 37 | 37 | 41 | 47 | 40 | 50 | 73 | 84 |
| D | 29 | 40 | 42 | 44 | 46 | 49 | 53 | 76 |
| E | 58 | 40 | 54 | 54 | 55 | 64 | 68 | 85 |

The placebos thus showed a slight reduction in keratisation on the 7th day as compared with the starting value; C - D, on the other hand, showed a considerable increase and are far above the value of 52% indicated by Lange for sound gingiva.

Example 2

Experiments were also carried out on guinea-pigs, viz. two test series with 9 animals each:

(a) for 7 days (b) for 21 days.

Each morning before feeding, a little sponge, which had been soaked in the test product for 1 minute, was pressed on to the inner right side of the oral mucous membrane of each test animal for a period of 2 minutes. The guinea pigs were treated as follows with the undermentioned products:

| Test Number | Solution | a-Series: 7 days; number of animals | b-Series: 21 days; number of animals |
|---|---|---|---|
| 1 | 0.5% aqueous solution of epigallo-catechin-3-gallate | 2 | 2 |
| 2 | 0.75% do. | 2 | 2 |
| 3 | 4.5% solution of zinc glycyrrhizinate in 100%-ethanol | 2 | - |
| 4 | 4.5% do. in 25%-ethanol | - | 2 |
| 5 | 4.5% do. in 10%-ethanol | - | 2 |
| 6 | Ethanol, 100% | 2 | - |
| | Control | 1 | 1 |

After completion of the test series, a microtome was used to make cutting preparations from the mouth and cheek parts, which had been fixed with formalin and coloured with hemalaun-eosin. The thickness of the horny layer and that of the epithelium layer were calculated in percentages for control. Basis:

4 mm* horn thickness control = 100%
32 mm* epithelium thickness control = 100%

* in mm after projection: 1 mm ≙ 2 x $10^{-3}$ mm in the preparation.

__Results__ : see page 7.

<u>Results:</u> Increase in thickness of the layers in %

Test Series (a) = 7 days

| No. | Test side | | Control side | |
|---|---|---|---|---|
| | Epithelium | Horn | Epithelium | Horn |
| 1 | 2 | 66 | 14 | 50 |
| 2 | -10 | 42 | 12 | 25 |
| 3 | 88 | 184 | - 4 | 42 |
| 4 | - | - | - | - |
| 5 | - | - | - | - |
| 6 | 16 | 80 | 32 | 58 |

Test Series (b) = 21 days

| No. | Test side | | Control side | |
|---|---|---|---|---|
| | Epithelium | Horn | Epithelium | Horn |
| 1 | 8 | 75 | -8 | 38 |
| 2 | 8 | 29 | 29 | 83 |
| 3 | - | - | - | - |
| 4 | 7 | 29 | 6 | 29 |
| 5 | 4 | 34 | -15 | 4 |
| 6 | - | - | - | - |

The animal tests confirm the results of the tests with humans, in which the use of the keratinising substances results in the thickness of the horny layer increasing many times more strongly than the thickness of the epithelium layer. The keratinising effect still lasts after 3 weeks.

Consequently, the active ingredients according to the invention are suitable for promoting the keratinisation of the human mucosa, in particular as additives to dentifrice compositions. Within the scope of prophylactic measures toothpastes, mouthwashes etc. in which these substances have been incorporated, can advantageously be used for the purpose of permanent oral hygiene.

## CLAIMS

1. A dentifrice composition containing from 0.1 to 10% by weight of a keratinisation-promoting agent selected from the group consisting of epigallocatechin-3-gallate, salts of polyvalent metals of glycyrrhizinic acid and mixtures thereof.

2. A composition according to claim 1, in which the keratinisation-promoting agent is zinc glycyrrhizinate.